# EUROPEAN PATENT APPLICATION

(11) **EP 4 570 293 A1**
(43) Date of publication of application: **18.06.2025**
(21) Application number: 22954801.1
(22) Date of filing: 16.11.2022
(51) Int. Cl.: A61M 25/01, A61B 34/30

(54) **CATHETER GUIDE WIRE DELIVERY METHOD, DRIVING APPARATUS, COMPUTER DEVICE, AND STORAGE MEDIUM**

(30) Priority: 08.08.2022 CN 202210945713
(71) Applicant: Shenzhen Institute of Advanced Biomedical Robot Co., Ltd., Shenzhen, Guangdong 518000 (CN)
(72) Inventor: YANG, Weinan, Shenzhen, Guangdong 518000 (CN); REN, Wenyong, Shenzhen, Guangdong 518000 (CN)
(74) Representative: Monteiro Alves, Inês
(86) International application number: PCT/CN2022/132347
(87) International publication number: WO 2024/031862

(57) **Abstract**

A driving apparatus for delivering a catheter guide wire includes a first driving mechanism, a second driving mechanism and a third driving mechanism. **In** the initial delivery stage, a catheter between the first driving mechanism and the second driving mechanism is in a bent state; during the first stage of delivery, the first driving mechanism delivers the catheter and a guide wire together, and in the continuous delivery process, the bending degree of the catheter between the first driving mechanism and the second driving mechanism gradually decreases; during the second stage of delivery, a detection mechanism detects whether the catheter between the first driving mechanism and the second driving mechanism is straightened, and if so, the third driving mechanism drives the second driving mechanism to move in the delivery direction of the catheter and the guide wire.

## Description

The present application claims priority to Chinese Patent Application No. 202210945713.X, filed with the China National Intellectual Property Administration on August 8, 2022 and entitled "METHOD AND DRIVING APPARATUS FOR DELIVERING CATHETER AND GUIDEWIRE", which is incorporated herein by reference in its entirety.

### TECHNICAL FIELD

The present application relates to the field of driving system technologies, and in particular, to a method and driving apparatus for delivering a catheter and a guidewire, a computer device, and a storage medium.

### BACKGROUND

An existing driving system for delivering a catheter and a guidewire clamps two ends of the catheter respectively through a first driving apparatus and a second driving apparatus, and then the catheter is delivered through displacement of the two driving apparatuses. The inventor realized that the catheter needs to be kept straightened all the time in the process described above, and therefore a distance between the first driving apparatus and the second driving apparatus needs to be consistent with a length of the catheter. Thus, an overall volume of the device is too large and bulky.

### SUMMARY

### TECHNICAL PROBLEMS

The main objective of the present application is to provide a method and driving apparatus for delivering a catheter and a guidewire, a computer device, and a storage medium, to resolve the technical problems in the existing technologies that the distance between the first driving apparatus and the second driving apparatus needs to be consistent with the length of the catheter, and the overall volume of the device is too large and bulky.

### TECHNICAL SOLUTIONS

A method for delivering a catheter and a guidewire provided in the present application is applied to a driving apparatus for delivering a catheter and a guidewire, and the driving apparatus includes a first driving mechanism, a second driving mechanism, a third driving mechanism, and a detection mechanism, where the method includes the following specific steps.

S1: The guidewire is threaded in the catheter, where a distance by which one end of the guidewire is exposed to one end of the catheter is a preset distance, one end of the catheter is clamped by the first driving mechanism, and the other end of the catheter is clamped by the second driving mechanism, where the catheter between the first driving mechanism and the second driving mechanism is bent.

S2: The first driving mechanism delivers the catheter and the guidewire together, where a bending degree of the catheter between the first driving mechanism and the second driving mechanism gradually decreases during continuous delivery.

S3: The detection mechanism detects whether the catheter between the first driving mechanism and the second driving mechanism is straightened, if so, the third driving mechanism drives the second driving mechanism to move in a delivery direction of the catheter and the guidewire, and the first driving mechanism and the second driving mechanism coordinate to deliver the catheter and the guidewire together, where the catheter between the first driving mechanism and the second driving mechanism is kept straightened during continuous delivery.

Further, the second driving mechanism includes a guidewire rotating assembly and a guidewire delivery assembly, the guidewire is rotated by the guidewire rotating assembly, and the guidewire is delivered by the guidewire delivery assembly.

Further, the first driving mechanism includes a catheter delivery assembly, and the catheter delivery assembly delivers the catheter and the guidewire together during the delivery in S2.

Further, during the delivery in S3, the third driving mechanism drives the second driving mechanism to move, and the catheter delivery assembly delivers one end of the catheter at the same speed as the second driving mechanism delivers the other end of the catheter, so as to keep the catheter between the first driving mechanism and the second driving mechanism straightened.

Further, the detection mechanism is a pressure sensor, and in S3, at a moment when the catheter is straightened, the pressure sensor detects a pressure signal and triggers the third driving mechanism to drive the second driving mechanism to move.

Further, during the continuous delivery in S3, the pressure sensor continuously detects the pressure signal to ensure that the catheter between the first driving mechanism and the second driving mechanism is straightened.

Further, the first driving mechanism further includes a first catheter rotating assembly, the second driving mechanism further includes a second catheter rotating assembly, and during the delivery in S3, the first catheter rotating assembly and the second catheter rotating assembly coordinate to rotate the catheter.

Further, the driving apparatus further includes a displacement detection apparatus, and the method further includes the following step.

S4: The displacement detection apparatus detects whether the second driving mechanism reaches a preset limit position; if so, the second driving mechanism stops displacement.

Further, the displacement detection apparatus is a photoelectric switch, and in S4, the photoelectric switch detects a moment when the second driving mechanism reaches the preset limit position, and the photoelectric switch detects a signal and triggers the second driving mechanism to stop moving.

Further, the first driving mechanism is fixedly arranged, and the third driving mechanism drives the second driving mechanism to move towards the first driving mechanism.

The present application further provides a driving apparatus for delivering a catheter and a guidewire, configured to implement the method for delivering a catheter and a guidewire, where the driving apparatus includes:
a first driving mechanism, configured to clamp one end of the catheter;
a second driving mechanism, configured to clamp the other end of the catheter;
a third driving mechanism, configured to drive the second driving mechanism to move in a delivery direction of the catheter and the guidewire; and
a detection mechanism, configured to detect whether the catheter between the first driving mechanism and the second driving mechanism is straightened; where
in an initial stage, the guidewire is threaded in the catheter, a distance by which one end of the guidewire is exposed to one end of the catheter is a preset distance, one end of the catheter is clamped by the first driving mechanism, and the other end of the catheter is clamped by the second driving mechanism, where the catheter between the first driving mechanism and the second driving mechanism is bent; in a first delivery stage, the first driving mechanism delivers the catheter and the guidewire together, where a bending degree of the catheter between the first driving mechanism and the second driving mechanism gradually decreases during continuous delivery; and in a second delivery stage, the detection mechanism detects whether the catheter between the first driving mechanism and the second driving mechanism is straightened, if so, the third driving mechanism drives the second driving mechanism to move in the delivery direction of the catheter and the guidewire, and the first driving mechanism and the second driving mechanism coordinate to deliver the catheter and the guidewire together, where the catheter between the first driving mechanism and the second driving mechanism is kept straightened during continuous delivery.

The present application further provides a computer device, including a memory and a processor, where the memory stores a computer program, and when the processor executes the computer program, a method for delivering a catheter and a guidewire is implemented, the method is applied to a driving apparatus for delivering a catheter and a guidewire, and the driving apparatus includes a first driving mechanism, a second driving mechanism, a third driving mechanism, and a detection mechanism, where the method includes the following specific steps.

S1: The guidewire is threaded in the catheter, where a distance by which one end of the guidewire is exposed to one end of the catheter is a preset distance, one end of the catheter is clamped by the first driving mechanism, and the other end of the catheter is clamped by the second driving mechanism, where the catheter between the first driving mechanism and the second driving mechanism is bent.

S2: The first driving mechanism delivers the catheter and the guidewire together, where a bending degree of the catheter between the first driving mechanism and the second driving mechanism gradually decreases during continuous delivery.

S3: The detection mechanism detects whether the catheter between the first driving mechanism and the second driving mechanism is straightened, if so, the third driving mechanism drives the second driving mechanism to move in a delivery direction of the catheter and the guidewire, and the first driving mechanism and the second driving mechanism coordinate to deliver the catheter and the guidewire together, where the catheter between the first driving mechanism and the second driving mechanism is kept straightened during continuous delivery.

The present application further provides a computer-readable storage medium, having a computer program stored thereon, where when the computer program is executed by a processor, a method for delivering a catheter and a guidewire is implemented, the method is applied to a driving apparatus for delivering a catheter and a guidewire, and the driving apparatus includes a first driving mechanism, a second driving mechanism, a third driving mechanism, and a detection mechanism, where the method includes the following specific steps.

S1: The guidewire is threaded in the catheter, where a distance by which one end of the guidewire is exposed to one end of the catheter is a preset distance, one end of the catheter is clamped by the first driving mechanism, and the other end of the catheter is clamped by the second driving mechanism, where the catheter between the first driving mechanism and the second driving mechanism is bent.

S2: The first driving mechanism delivers the catheter and the guidewire together, where a bending degree of the catheter between the first driving mechanism and the second driving mechanism gradually decreases during continuous delivery.

S3: The detection mechanism detects whether the catheter between the first driving mechanism and the second driving mechanism is straightened, if so, the third driving mechanism drives the second driving mechanism to move in a delivery direction of the catheter and the guidewire, and the first driving mechanism and the second driving mechanism coordinate to deliver the catheter and the guidewire together, where the catheter between the first driving mechanism and the second driving mechanism is kept straightened during continuous delivery.

### BENEFICIAL EFFECTS

Compared with the existing technologies, the present application provides a method and driving apparatus for delivering a catheter and a guidewire, a computer device, and a storage medium. During delivery of the catheter, the driving apparatus for delivering a catheter and a guidewire allows the catheter between the first driving mechanism and the second driving mechanism to be bent, so that a distance between the first driving mechanism and the second driving mechanism is greatly shortened, an overall size of the driving apparatus is reduced, and occupied space and overall mass of the driving apparatus are reduced.

### BRIEF DESCRIPTION OF DRAWINGS

FIG. 1 is a schematic flowchart of a method for delivering a catheter and a guidewire according to the present application;
FIG. 2 is a schematic diagram of a state in step S1 according to the present application;
FIG. 3 is a schematic diagram I of a state of a first delivery stage according to the present application;
FIG. 4 is a schematic diagram II of a state of the first delivery stage according to the present application;
FIG. 5 is a schematic diagram I of a state of a second delivery stage according to the present application;
FIG. 6 is a schematic diagram II of a state of the second delivery stage according to the present application;
FIG. 7 is a schematic diagram III of a state of the second delivery stage according to the present application; and
FIG. 8 is a schematic diagram of a structure of a driving apparatus according to the present application.

Names of components marked in the figures are given below: 1. first driving mechanism; 11. catheter delivery assembly; 111. roller; 12. first catheter rotating assembly; 2. second driving mechanism; 21. guidewire rotating assembly; 22. guidewire delivery assembly; 23. second catheter rotating assembly; 3. third driving mechanism; 4. detection mechanism; 5. catheter; 6. guidewire; 7. clamping block; 8. connecting plate; 9. displacement detection apparatus.

The implementation of objectives, functional features, and advantages of the present application will be further described with reference to the embodiments and accompanying drawings.

### PREFERRED IMPLEMENTATIONS OF THE PRESENT INVENTION

It should be understood that specific embodiments described herein are merely intended to explain the present application, but not intended to limit the present application.

The following clearly and completely describes the technical solutions in embodiments of the present application with reference to the accompanying drawings in the embodiments of the present application. Clearly, the described embodiments are merely some rather than all of the embodiments of the present application. Based on the embodiments of the present application, all other embodiments derived by a person of ordinary skill in the art without creative efforts should fall within the protection scope of the present application.

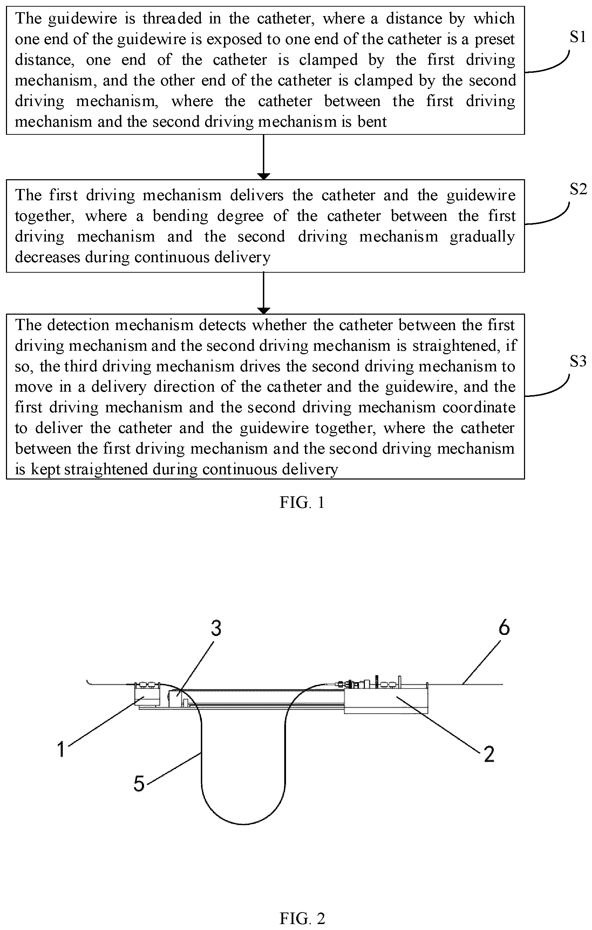

Referring to FIG. 1, a method for delivering a catheter and a guidewire according to an embodiment of the present application is applied to a driving apparatus for delivering a catheter and a guidewire, and the driving apparatus includes a first driving mechanism 1, a second driving mechanism 2, a third driving mechanism 3, and a detection mechanism 4, where the method includes the following specific steps.

S1: The guidewire 6 is threaded in the catheter 5, where a distance by which one end of the guidewire 6 is exposed to one end of the catheter 5 is a preset distance, one end of the catheter 5 is clamped by the first driving mechanism 1, and the other end of the catheter 5 is clamped by the second driving mechanism 2, where the catheter 5 between the first driving mechanism 1 and the second driving mechanism 2 is bent.

As shown in FIG. 2, step S1 is an initial stage in which the guidewire 6 is first threaded in the catheter 5. The guidewire 6 may be threaded in the catheter 5 manually, by using a mechanical device, or by other means. During delivery of the catheter and the guidewire, one end of the guidewire 6 needs to be exposed to the catheter 5 by a distance, and the distance by which the end of the guidewire 6 is exposed to the end of the catheter 5 is adjusted to a preset distance manually, by using a mechanical device, or by other means. After the guidewire 6 and the catheter 5 are arranged, one end of the catheter 5 is clamped by the first driving mechanism 1, and the other end of the catheter 5 is clamped by the second driving mechanism 2. It should be noted here that the catheter 5 between the first driving mechanism 1 and the second driving mechanism 2 is bent in this case. Because the catheter 5 is bent in the initial stage, a distance between the first driving mechanism 1 and the second driving mechanism 2 can be greatly shortened.

S2: The first driving mechanism 1 delivers the catheter 5 and the guidewire 6 together, where a bending degree of the catheter 5 between the first driving mechanism 1 and the second driving mechanism 2 gradually decreases during continuous delivery.

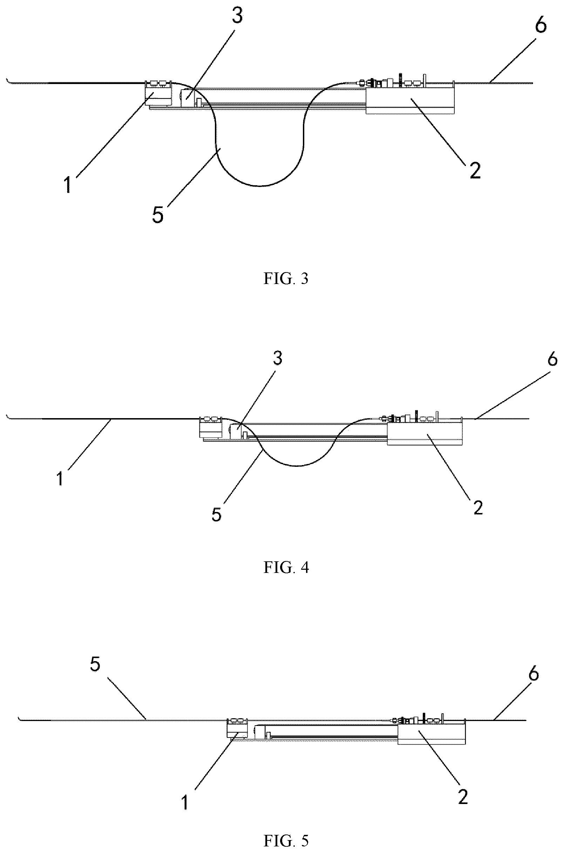

As shown in FIG. 3 and FIG. 4, step S2 is a first delivery stage. In this stage, the catheter 5 is always kept bent. However, during continuous delivery of the catheter 5, the bending degree of the catheter 5 gradually decreases.

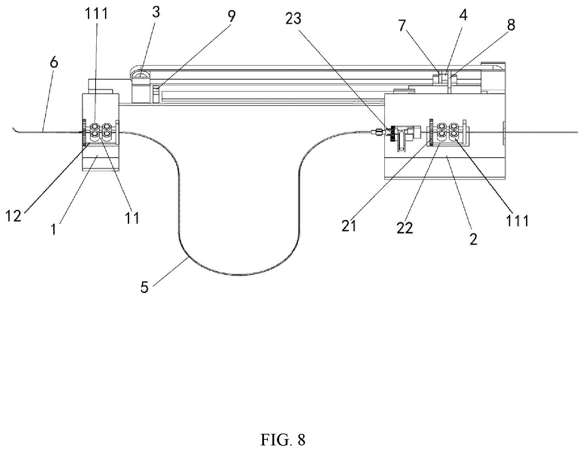

Further, referring to FIG. 8, the first driving mechanism 1 includes a catheter delivery assembly 11, and the catheter delivery assembly 11 delivers the catheter 5 and the guidewire 6 together during the delivery in S2. The catheter delivery assembly 11 includes a plurality of rotatable rollers 111. The plurality of rollers 111 are arranged on both sides of the catheter 5 to deliver the catheter 5 through rotation of the rollers 111 themselves under the action of friction between the rollers 111 and the catheter 5. The above is only one of the ways in which the catheter rotating assembly may deliver the catheter 5, and other alternative ways in which the catheter 5 can be delivered are within the protection scope of the present application.

S3: The detection mechanism 4 detects whether the catheter 5 between the first driving mechanism 1 and the second driving mechanism 2 is straightened, if so, the third driving mechanism 3 drives the second driving mechanism 2 to move in a delivery direction of the catheter 5 and the guidewire 6, and the first driving mechanism 1 and the second driving mechanism 2 coordinate to deliver the catheter 5 and the guidewire 6 together, where the catheter 5 between the first driving mechanism 1 and the second driving mechanism 2 is kept straightened during continuous delivery.

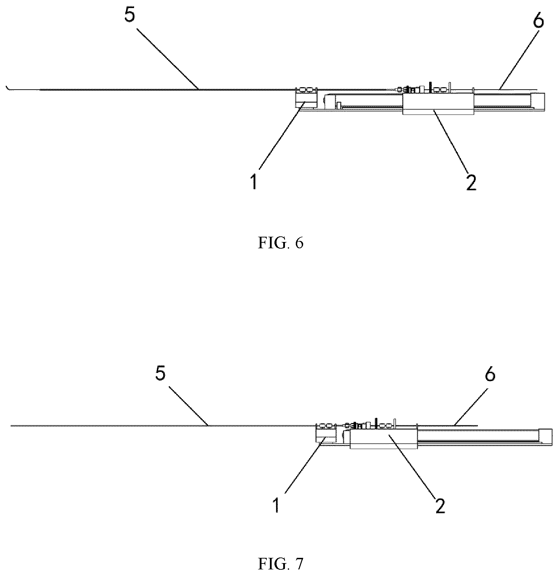

As shown in FIG. 5 to FIG. 7, step S3 is a second delivery stage. Because the bending degree of the catheter 5 gradually decreases with the first delivery stage, the catheter 5 will eventually be straightened. In this stage, a detection mechanism 4 needs to be provided to detect whether the catheter 5 becomes straightened. If so, the third driving mechanism 3 drives the second driving mechanism 2 to move in a delivery direction of the catheter 5 and the guidewire 6, the first driving mechanism 1 and the second driving mechanism 2 coordinate to deliver the catheter 5 and the guidewire 6 together, and the catheter 5 between the first driving mechanism 1 and the second driving mechanism 2 is kept straightened during continuous delivery.

Further, referring to FIG. 8, during the delivery in S3, the third driving mechanism 3 drives the second driving mechanism 2 to move, and the catheter delivery assembly 11 delivers one end of the catheter 5 at the same speed as the second driving mechanism 2 delivers the other end of the catheter 5, so as to keep the catheter 5 between the first driving mechanism 1 and the second driving mechanism 2 straightened. Specifically, the catheter delivery assembly 11 uses the rollers 111 to deliver one end of the catheter 5, so that the speed of the catheter 5 is determined by a rotational speed of the rollers 111. The speed at which the second driving mechanism 2 delivers the other end of the catheter 5 is determined by a displacement speed of the second driving mechanism 2. Because the displacement of the second driving mechanism 2 is driven by the third driving mechanism 3, a speed at which the third driving mechanism 3 drives the second driving mechanism 2 should be consistent with the speed at which the rollers 111 deliver one end of the catheter 5. In some feasible implementations, the third driving mechanism 3 is of a synchronous belt transmission structure or a screw transmission structure. The above is only two ways in which the third driving mechanism 3 may drive the first driving mechanism 1 to displace, and other alternative ways in which the first driving mechanism 1 can be displaced are within the protection scope of the present application.

Further, referring to FIG. 5 and FIG. 8, the detection mechanism 4 is a pressure sensor, and in S3, at a moment when the catheter 5 is straightened, the pressure sensor detects a pressure signal and triggers the third driving mechanism 3 to drive the second driving mechanism 2 to move. One end of the pressure sensor is mounted on a clamping block 7, and the other end is in contact with a connecting plate 8. At a moment when the catheter 5 between the first driving mechanism 1 and the second driving mechanism 2 is straightened, a delivery force of the first driving mechanism 1 on the catheter 5 is transmitted through a rear end of the catheter 5 to the second driving mechanism 2, so as to pull the second driving mechanism 2 to move, and concurrently drive the connecting plate 8 to move. The connecting plate 8 generates a pressure on a surface of the pressure sensor during movement, and the pressure sensor detects a pressure signal and triggers the second driving mechanism 2 to move.

Further, referring to FIG. 5 to FIG. 8, during the continuous delivery in S3, the pressure sensor continuously detects the pressure signal to ensure that the catheter 5 between the first driving mechanism 1 and the second driving mechanism 2 is straightened. In an existing driving apparatus, when the catheter 5 is bent, there is no mechanism to detect whether the catheter 5 is bent or straightened, so that a user needs to keep an eye on the state of the catheter 5 all the time, which affects delivery efficiency of the catheter 5. The pressure sensor automatically detects whether the catheter 5 is bent or straightened, and automatically switches to a corresponding delivery method when the state changes, so that the user does not need to keep an eye on the state of the catheter 5 all the time, and the delivery efficiency of the catheter 5 is improved.

Further, referring to FIG. 8, the first driving mechanism 1 further includes a first catheter rotating assembly 12, the second driving mechanism 2 further includes a second catheter rotating assembly 23, and during the delivery in S3, the first catheter rotating assembly 12 and the second catheter rotating assembly 23 coordinate to rotate the catheter 5. In the second delivery stage, with the catheter 5 straightened, the catheter 5 can be rotated to deliver the catheter 5 to a limit distance. The catheter delivery assembly 11 includes a plurality of rotatable rollers 111, and the rollers 111 are arranged on the first catheter rotating assembly 12. The first catheter rotating assembly 12 drives, through a gear, the rollers 111 to revolve around an axis of the catheter 5 to rotate the catheter 5. The second catheter rotating assembly 23 includes a T-valve that is provided with a rotating component, and a rear end of the catheter 5 is connected to the rotating component. When the catheter 5 needs to be rotated, the first catheter rotating assembly 12 drives one end of the catheter 5 to rotate, and the rotating component drives the other end of the catheter 5 to rotate synchronously.

Further, referring to FIG. 8, the driving apparatus further includes a displacement detection apparatus 9, and the method further includes the following step.

S4: The displacement detection apparatus detects whether the second driving mechanism reaches a preset limit position; if so, the second driving mechanism 2 stops displacement. Specifically, the displacement detection apparatus 4 is a photoelectric switch, and in S4, the photoelectric switch detects a moment when the second driving mechanism reaches the preset limit position, and the photoelectric switch detects a signal and triggers the second driving mechanism 2 to stop moving. In the second delivery stage, the catheter 5 continues to be delivered, and the second driving mechanism 2 gradually approaches the first driving mechanism 1. At a moment when the second driving mechanism moves to the limit distance, the photoelectric switch detects a signal, and the second delivery stage ends. With the photoelectric switch, an operating state of the second driving mechanism 2 can be quickly controlled, thereby effectively preventing the second driving mechanism 2 from damaging other components during operation.

Further, referring to FIG. 8, the second driving mechanism 2 includes a guidewire rotating assembly 21 and a guidewire delivery assembly 22, the guidewire 6 is rotated by the guidewire rotating assembly 21, and the guidewire 6 is delivered by the guidewire delivery assembly 22. When the catheter 5 and the guidewire 6 enter a conduit and come across a branch in the conduit, if the guidewire 6 is delivered only by the guidewire delivery assembly 22, the guidewire 6 may not pass smoothly or may be broken because a bending angle of the conduit is too sharp and a bending length is too short. In this case, the guidewire needs to be pushed by the guidewire delivery assembly 22 to have a displacement, and meanwhile, the guidewire rotating assembly 21 rotates the guidewire 6, and then delivers the guidewire 6 to a position. Specifically, the guidewire delivery assembly 22 includes a plurality of rotatable rollers 111. The plurality of rollers 111 are arranged on both sides of the guidewire 6 to deliver the guidewire 6 through rotation of the rollers 111 themselves under the action of friction between the rollers 111 and the guidewire 6. The guidewire rotating assembly 21 drives, through a gear, the rollers 111 to revolve around an axis of the guidewire 6 to rotate the guidewire 6. The above is only one of the ways in which the guidewire rotating assembly 21 and the guidewire delivery assembly 22 may rotate or deliver the guidewire 6, and other alternative ways in which the guidewire 6 can be rotated or delivered are within the protection scope of the present application.

Further, referring to FIG. 8, the first driving mechanism 1 is fixedly arranged, and the third driving mechanism 3 drives the second driving mechanism 2 to move towards the first driving mechanism 1. In the existing technologies, the catheter 5 needs to be delivered through coordinated movement of a plurality of driving mechanisms. During delivery of the catheter 5, in order to ensure that the plurality of driving mechanisms have sufficient motion strokes in the delivery direction of the catheter 5, the driving apparatus should have enough internal space. Thus, an overall volume of the driving apparatus is too large and bulky, which is unfavorable for spatial layout of the driving apparatus. Because the first driving mechanism 1 is fixedly arranged, and only the second driving mechanism 2 is displaced on a frame, an overall size of a slave end is reduced, and occupied space and overall mass of the driving apparatus are reduced through the above-mentioned reasonable structural design, which is favorable for spatial layout of the driving apparatus.

The present application further provides a driving apparatus for delivering a catheter and a guidewire, configured to implement the method for delivering a catheter and a guidewire, where the driving apparatus includes:
a first driving mechanism 1, configured to clamp one end of the catheter 5;
a second driving mechanism 2, configured to clamp the other end of the catheter 5;
a third driving mechanism 3, configured to drive the second driving mechanism 2 to move in a delivery direction of the catheter 5 and the guidewire 6; and
a detection mechanism 4, configured to detect whether the catheter 5 between the first driving mechanism 1 and the second driving mechanism 2 is straightened; where
in an initial stage, the guidewire 6 is threaded in the catheter 5, a distance by which one end of the guidewire 6 is exposed to one end of the catheter 5 is a preset distance, one end of the catheter 5 is clamped by the first driving mechanism 1, and the other end of the catheter 5 is clamped by the second driving mechanism 2, where the catheter 5 between the first driving mechanism 1 and the second driving mechanism 2 is bent; in a first delivery stage, the first driving mechanism 1 delivers the catheter 5 and the guidewire 6 together, where a bending degree of the catheter 5 between the first driving mechanism 1 and the second driving mechanism 2 gradually decreases during continuous delivery; and in a second delivery stage, the detection mechanism 4 detects whether the catheter 5 between the first driving mechanism 1 and the second driving mechanism 2 is straightened, if so, the third driving mechanism 3 drives the second driving mechanism 2 to move in the delivery direction of the catheter 5 and the guidewire 6, and the first driving mechanism 1 and the second driving mechanism 2 coordinate to deliver the catheter 5 and the guidewire 6 together, where the catheter 5 between the first driving mechanism 1 and the second driving mechanism 2 is kept straightened during continuous delivery.

The present application further provides a computer device, including a memory and a processor, where the memory stores a computer program, and when the processor executes the computer program, a method for delivering a catheter and a guidewire is implemented, the method is applied to a driving apparatus for delivering a catheter and a guidewire, and the driving apparatus includes a first driving mechanism 1, a second driving mechanism 2, a third driving mechanism 3, and a detection mechanism 4, where the method includes the following specific steps.

S1: The guidewire 6 is threaded in the catheter 5, where a distance by which one end of the guidewire 6 is exposed to one end of the catheter 5 is a preset distance, one end of the catheter 5 is clamped by the first driving mechanism 1, and the other end of the catheter 5 is clamped by the second driving mechanism 2, where the catheter 5 between the first driving mechanism 1 and the second driving mechanism 2 is bent.

S2: The first driving mechanism 1 delivers the catheter 5 and the guidewire 6 together, where a bending degree of the catheter 5 between the first driving mechanism 1 and the second driving mechanism 2 gradually decreases during continuous delivery.

S3: The detection mechanism 4 detects whether the catheter 5 between the first driving mechanism 1 and the second driving mechanism 2 is straightened, if so, the third driving mechanism 3 drives the second driving mechanism 2 to move in a delivery direction of the catheter 5 and the guidewire 6, and the first driving mechanism 1 and the second driving mechanism 2 coordinate to deliver the catheter 5 and the guidewire 6 together, where the catheter 5 between the first driving mechanism 1 and the second driving mechanism 2 is kept straightened during continuous delivery.

The present application further provides a computer-readable storage medium, having a computer program stored thereon, where when the computer program is executed by a processor, a method for delivering a catheter and a guidewire is implemented, the method is applied to a driving apparatus for delivering a catheter and a guidewire, and the driving apparatus includes a first driving mechanism 1, a second driving mechanism 2, a third driving mechanism 3, and a detection mechanism 4, where the method includes the following specific steps.

S1: The guidewire 6 is threaded in the catheter 5, where a distance by which one end of the guidewire 6 is exposed to one end of the catheter 5 is a preset distance, one end of the catheter 5 is clamped by the first driving mechanism 1, and the other end of the catheter 5 is clamped by the second driving mechanism 2, where the catheter 5 between the first driving mechanism 1 and the second driving mechanism 2 is bent.

S2: The first driving mechanism 1 delivers the catheter 5 and the guidewire 6 together, where a bending degree of the catheter 5 between the first driving mechanism 1 and the second driving mechanism 2 gradually decreases during continuous delivery.

S3: The detection mechanism 4 detects whether the catheter 5 between the first driving mechanism 1 and the second driving mechanism 2 is straightened, if so, the third driving mechanism 3 drives the second driving mechanism 2 to move in a delivery direction of the catheter 5 and the guidewire 6, and the first driving mechanism 1 and the second driving mechanism 2 coordinate to deliver the catheter 5 and the guidewire 6 together, where the catheter 5 between the first driving mechanism 1 and the second driving mechanism 2 is kept straightened during continuous delivery.

A person of ordinary skill in the art may understand that all or some of the processes of the methods in the foregoing embodiments may be implemented by a computer program instructing related hardware. The computer program may be stored in a non-volatile computer-readable storage medium. When the computer program is executed, the processes in the foregoing method embodiments may be included. Any references to a memory, storage, a database or another medium provided in the present application and used in the embodiments may include a non-volatile memory and/or a volatile memory. The non-volatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external cache memory. By way of illustration rather than limitation, the RAM is available in various forms, such as a static RAM (SRAM), a dynamic RAM (DRAM), a synchronous DRAM (SDRAM), a double data rate SDRAM (SSRSDRAM), an enhanced SDRAM (ESDRAM), a synchronization link (Synchlink) DRAM (SLDRAM), a memory bus (Rambus) direct RAM (RDRAM), a direct rambus dynamic RAM (DRDRAM), and a rambus dynamic RAM (RDRAM).

It should be noted that the term "include", "comprise", or any other variant thereof as used herein is intended to cover a non-exclusive inclusion, so that a process, apparatus, article, or method that includes a series of elements includes not only those elements but also other elements that are not expressly listed, or includes elements inherent to such a process, apparatus, article, or method. Without further limitation, the element defined by the sentence "including a" does not exclude that other identical elements also exist in the process, apparatus, article, or method including the element.

The above description is only preferred embodiments of the present application, and thus does not limit the scope of the patent of the present application. Any equivalent structure transformation or equivalent process transformation that is made based on the specification and accompanying drawings of the present application, or that is directly or indirectly applied to other related technical fields, is equally included in the protection scope of the patent of the present application.

## Claims

1. A method for delivering a catheter and a guidewire, applied to a driving apparatus for delivering a catheter and a guidewire, the driving apparatus comprising a first driving mechanism, a second driving mechanism, a third driving mechanism, and a detection mechanism, wherein the method comprises the following specific steps:
S1: threading the guidewire in the catheter, wherein a distance by which one end of the guidewire is exposed to one end of the catheter is a preset distance, one end of the catheter is clamped by the first driving mechanism, and the other end of the catheter is clamped by the second driving mechanism, wherein the catheter between the first driving mechanism and the second driving mechanism is bent;
S2: delivering, by the first driving mechanism, the catheter and the guidewire together, wherein a bending degree of the catheter between the first driving mechanism and the second driving mechanism gradually decreases during continuous delivery; and
S3: detecting, by the detection mechanism, whether the catheter between the first driving mechanism and the second driving mechanism is straightened, if so, driving, by the third driving mechanism, the second driving mechanism to move in a delivery direction of the catheter and the guidewire, and coordinating, by the first driving mechanism and the second driving mechanism, to deliver the catheter and the guidewire together, wherein the catheter between the first driving mechanism and the second driving mechanism is kept straightened during continuous delivery.

2. The method for delivering a catheter and a guidewire according to claim 1, wherein the second driving mechanism comprises a guidewire rotating assembly and a guidewire delivery assembly, the guidewire is rotated by the guidewire rotating assembly, and the guidewire is delivered by the guidewire delivery assembly.

3. The method for delivering a catheter and a guidewire according to claim 1, wherein the first driving mechanism comprises a catheter delivery assembly, and the catheter delivery assembly delivers the catheter and the guidewire together during the delivery in S2.

4. The method for delivering a catheter and a guidewire according to claim 3, wherein during the delivery in S3, the third driving mechanism drives the second driving mechanism to move, and the catheter delivery assembly delivers one end of the catheter at the same speed as the second driving mechanism delivers the other end of the catheter, so as to keep the catheter between the first driving mechanism and the second driving mechanism straightened.

5. The method for delivering a catheter and a guidewire according to claim 1, wherein the detection mechanism is a pressure sensor, and in S3, at a moment when the catheter is straightened, the pressure sensor detects a pressure signal and triggers the third driving mechanism to drive the second driving mechanism to move.

6. The method for delivering a catheter and a guidewire according to claim 5, wherein during the continuous delivery in S3, the pressure sensor continuously detects the pressure signal to ensure that the catheter between the first driving mechanism and the second driving mechanism is straightened.

7. The method for delivering a catheter and a guidewire according to claim 1, wherein the first driving mechanism further comprises a first catheter rotating assembly, the second driving mechanism further comprises a second catheter rotating assembly, and during the delivery in S3, the first catheter rotating assembly and the second catheter rotating assembly coordinate to rotate the catheter.

8. The method for delivering a catheter and a guidewire according to claim 1, wherein the driving apparatus further comprises a displacement detection apparatus, and the method further comprises:
S4: detecting, by the displacement detection apparatus, whether the second driving mechanism reaches a preset limit position; if so, stopping, by the second driving mechanism, displacement.

9. The method for delivering a catheter and a guidewire according to claim 8, wherein the displacement detection apparatus is a photoelectric switch, and in S4, the photoelectric switch detects a moment when the second driving mechanism reaches the preset limit position, and the photoelectric switch detects a signal and triggers the second driving mechanism to stop moving.

10. The method for delivering a catheter and a guidewire according to claim 1, wherein the first driving mechanism is fixedly arranged, and the third driving mechanism drives the second driving mechanism to move towards the first driving mechanism.

11. A driving apparatus for delivering a catheter and a guidewire, configured to implement the method for delivering a catheter and a guidewire according to any one of claims 1 to 10, wherein the driving apparatus comprises:
a first driving mechanism, configured to clamp one end of the catheter;
a second driving mechanism, configured to clamp the other end of the catheter;
a third driving mechanism, configured to drive the second driving mechanism to move in a delivery direction of the catheter and the guidewire; and
a detection mechanism, configured to detect whether the catheter between the first driving mechanism and the second driving mechanism is straightened; wherein
in an initial stage, the guidewire is threaded in the catheter, a distance by which one end of the guidewire is exposed to one end of the catheter is a preset distance, one end of the catheter is clamped by the first driving mechanism, and the other end of the catheter is clamped by the second driving mechanism, wherein the catheter between the first driving mechanism and the second driving mechanism is bent; in a first delivery stage, the first driving mechanism delivers the catheter and the guidewire together, wherein a bending degree of the catheter between the first driving mechanism and the second driving mechanism gradually decreases during continuous delivery; and in a second delivery stage, the detection mechanism detects whether the catheter between the first driving mechanism and the second driving mechanism is straightened, if so, the third driving mechanism drives the second driving mechanism to move in the delivery direction of the catheter and the guidewire, and the first driving mechanism and the second driving mechanism coordinate to deliver the catheter and the guidewire together, wherein the catheter between the first driving mechanism and the second driving mechanism is kept straightened during continuous delivery.

12. The driving apparatus for delivering a catheter and a guidewire according to claim 11, wherein the first driving mechanism comprises a catheter delivery assembly comprising a plurality of rotatable rollers, and the plurality of rollers are arranged on both sides of the catheter to deliver the catheter through rotation of the rollers themselves under the action of friction between the rollers and the catheter.

13. The driving apparatus for delivering a catheter and a guidewire according to claim 12, wherein
the first driving mechanism further comprises a first catheter rotating assembly, the rollers are arranged on the first catheter rotating assembly, and the first catheter rotating assembly drives, through a gear, the rollers to revolve around an axis of the catheter to rotate the catheter.

14. The driving apparatus for delivering a catheter and a guidewire according to claim 13, wherein
the second driving mechanism further comprises a second catheter rotating assembly, the second catheter rotating assembly comprises a T-valve that is provided with a rotating component, and a rear end of the catheter is connected to the rotating component.

15. The driving apparatus for delivering a catheter and a guidewire according to claim 11, wherein the second driving mechanism comprises a guidewire delivery assembly comprising a plurality of rotatable rollers, and the plurality of rollers are arranged on both sides of the guidewire to deliver the guidewire through rotation of the rollers themselves under the action of friction between the rollers and the guidewire.

16. The driving apparatus for delivering a catheter and a guidewire according to claim 11, wherein the third driving mechanism is of a synchronous belt transmission structure or a screw transmission structure.

17. The driving apparatus for delivering a catheter and a guidewire according to claim 11, wherein the detection mechanism is a pressure sensor, one end of the pressure sensor is in contact with a connecting plate, and at a moment when the catheter between the first driving mechanism and the second driving mechanism is straightened, a delivery force of the first driving mechanism on the catheter is transmitted through a rear end of the catheter to the second driving mechanism, so as to pull the second driving mechanism to move, and concurrently drive the connecting plate to move, the connecting plate generates a pressure on a surface of the pressure sensor during movement, and the pressure sensor detects a pressure signal and triggers the second driving mechanism to move.

18. A computer device, comprising a memory and a processor, wherein the memory stores a computer program, and when the processor executes the computer program, a method for delivering a catheter and a guidewire is implemented, the method is applied to a driving apparatus for delivering a catheter and a guidewire, and the driving apparatus comprises a first driving mechanism, a second driving mechanism, a third driving mechanism, and a detection mechanism, wherein the method comprises the following specific steps:
S1: threading the guidewire in the catheter, wherein a distance by which one end of the guidewire is exposed to one end of the catheter is a preset distance, one end of the catheter is clamped by the first driving mechanism, and the other end of the catheter is clamped by the second driving mechanism, wherein the catheter between the first driving mechanism and the second driving mechanism is bent;
S2: delivering, by the first driving mechanism, the catheter and the guidewire together, wherein a bending degree of the catheter between the first driving mechanism and the second driving mechanism gradually decreases during continuous delivery; and
S3: detecting, by the detection mechanism, whether the catheter between the first driving mechanism and the second driving mechanism is straightened, if so, driving, by the third driving mechanism, the second driving mechanism to move in a delivery direction of the catheter and the guidewire, and coordinating, by the first driving mechanism and the second driving mechanism, to deliver the catheter and the guidewire together, wherein the catheter between the first driving mechanism and the second driving mechanism is kept straightened during continuous delivery.

19. A computer-readable storage medium, having a computer program stored thereon, wherein when the computer program is executed by a processor, a method for delivering a catheter and a guidewire is implemented, the method is applied to a driving apparatus for delivering a catheter and a guidewire, and the driving apparatus comprises a first driving mechanism, a second driving mechanism, a third driving mechanism, and a detection mechanism, wherein the method comprises the following specific steps:
S1: threading the guidewire in the catheter, wherein a distance by which one end of the guidewire is exposed to one end of the catheter is a preset distance, one end of the catheter is clamped by the first driving mechanism, and the other end of the catheter is clamped by the second driving mechanism, wherein the catheter between the first driving mechanism and the second driving mechanism is bent;
S2: delivering, by the first driving mechanism, the catheter and the guidewire together, wherein a bending degree of the catheter between the first driving mechanism and the second driving mechanism gradually decreases during continuous delivery; and
S3: detecting, by the detection mechanism, whether the catheter between the first driving mechanism and the second driving mechanism is straightened, if so, driving, by the third driving mechanism, the second driving mechanism to move in a delivery direction of the catheter and the guidewire, and coordinating, by the first driving mechanism and the second driving mechanism, to deliver the catheter and the guidewire together, wherein the catheter between the first driving mechanism and the second driving mechanism is kept straightened during continuous delivery.
